# EUROPEAN PATENT APPLICATION

(11) **EP 4 299 084 A1**
(43) Date of publication of application: **03.01.2024**
(21) Application number: 21721962.5
(22) Date of filing: 24.02.2021
(51) Int. Cl.: A61M 1/00, C12M 1/00, A61B 17/00

(54) **DEVICE FOR PROCESSING FAT TISSUE AND METHOD FOR PROCESSING FAT TISSUE**

(71) Applicant: Leartiker, S.Coop., 48270 Markina-Xemein (Bizkaia) (ES); Clinique Ramsay Santé Belharra, 64100 Bayonne (FR); O.S. Frenos, S.L., 31170 Arazuri Navarra (ES)
(72) Inventor: LARREATEGI, Pablo, 48270 Markina-Xemein (ES); URTAZA, Uzuri, 48270 Markina-Xemein (ES); ZALDUA, Ane Miren, 48270 Markina-Xemein (ES); JOYEUX, Patrick, 64100 Bayonne (FR); OLLACARIZQUETA, Yvonne Teresa, 31192 Mutilva (ES)
(74) Representative: Igartua, Ismael
(86) International application number: PCT/ES2021/070130
(87) International publication number: WO 2022/180278

(57) **Abstract**

Device for the processing of fatty tissue of animal origin, the device being configured for separating by decantation a first waste phase and a second processed fatty tissue phase after mixing the fatty tissue with a washing solution, the device comprising a decantation chamber (1) comprising a decantation surface (11) where the fatty tissue and washing solution mixture is arranged and where separation by decantation takes place, and the device also comprising a waste extraction conduit (3) for extracting the waste phase and a filter (4) for preventing the passage of the processed fatty tissue phase through the waste extraction conduit (3), wherein the waste extraction conduit (3) is communicated with the decantation chamber (1) through an opening (30) of the decantation chamber (1), the filter (4) is housed in the waste extraction conduit (3), and when the device is in a waste extraction position, the waste extraction conduit (3) is arranged below the decantation chamber (1). Method for processing fatty tissue of animal origin.

## Description

### TECHNICAL FIELD

The present invention relates to devices and methods for the collection and processing of fatty tissue of animal origin.

### PRIOR ART

Transplantations of autologous fatty tissue grafts in human body shaping processes, for example, in breast reconstruction for people who have undergone a mastectomy, are becoming increasingly more common. Autologous fat tissue must be processed or washed before transplantation to prevent rejection by the patient. The time from the extraction of the fatty tissue to the moment of its transplantation must be as short as possible as it directly influences transplantation success, greatly reducing the probability of rejection of the fatty tissue graft. Fatty tissue is rather delicate, so it is of interest to process it with the smallest number of steps possible, and if possible, in closed circuits to minimize exposure to outside air to the furthest extent.

US8366694B1 describes a device for the processing of a fatty tissue extracted from a patient by means of decantation and vacuum application processes comprising a decantation area where an extracted fatty tissue and washing solution mixture is arranged, and where separation of the fatty tissue from the washing solution takes place, a processed fatty tissue phase and a waste phase being generated. The waste phase comprises the washing solution along with traces of blood and other cells present in the fatty tissue to be processed, the removal of which from the processed fatty tissue is of interest. The device comprises a filter inside the decantation area which generates a first chamber where the separated fatty tissue is arranged and a second chamber where the waste solution is arranged during the decantation and vacuum application process.

### DISCLOSURE OF THE INVENTION

The object of the invention is to provide a device for the processing of fatty tissue of animal origin, a method for processing fatty tissue of animal origin, and the use of the device for the processing of fatty tissue, as defined in the claims.

One aspect of the invention relates to a device for the processing of fatty tissue of animal origin, the device being configured for separating by decantation a first waste phase and a second processed fatty tissue phase after mixing the fatty tissue with a washing solution, the device comprising a decantation chamber comprising a decantation surface where the fatty tissue and washing solution mixture is arranged and where separation by decantation takes place, and the device also comprising a waste extraction conduit for extracting the waste phase and a filter for preventing the passage of the processed fatty tissue phase through the waste extraction conduit, wherein the waste extraction conduit is communicated with the decantation chamber through an opening of the decantation chamber, the filter is housed in the waste extraction conduit, and when the device is in a waste extraction position, the waste extraction conduit is arranged below the decantation chamber.

Another aspect of the invention relates to a method for processing fatty tissue of animal origin, comprising the following steps:
- a step of mixing an amount of fatty tissue of animal origin and a washing solution in a decantation chamber;
- a step of separating by decantation a first waste phase and a second processed fatty tissue phase in the decantation chamber;
- a step of extracting the waste phase from the decantation chamber through a waste extraction conduit; and
- a step of extracting the second processed fatty tissue phase from the decantation chamber.

In the method of the invention, the waste phase is filtered during the step of extracting the waste phase through the waste extraction conduit.

Another aspect of the invention relates to the use of the device of the invention for the processing of fatty tissue.

Since the fatty tissue is processed by means of a process involving separation by decantation, in which the main force is the gravitational force, and since the fatty tissue to be processed is arranged in a single chamber without the presence of filters which generate different chambers, the decantation and therefore the separation process is expedited without damaging the cellular content of the fatty tissue. The device and method of the invention allow processing fatty tissues in a matter of minutes, optionally under sterile conditions, where the extraction of fatty tissue, the processing of fatty tissue, and the transplantation in patients during the operation process in the operating room are allowed.

These and other advantages and features of the invention will become apparent in view of figures and detailed description of the invention.

### DESCRIPTION OF THE DRAWINGS

Figure 1 shows a schematic depiction of the device according to an embodiment of the invention in a decantation and extraction position of waste phase.
Figure 2 shows the schematic depiction of Figure 1 in a processed fatty tissue phase extraction position.
Figure 3 shows a depiction of the device for the processing of fatty tissue of animal origin according to another embodiment of the invention.
Figure 4 shows a depiction of the device for the processing of fatty tissue of animal origin according to another embodiment of the invention.
Figure 5 shows a schematic depiction of the steps of an embodiment of the method for processing fatty tissue of animal origin of the invention.

### DETAILED DISCLOSURE OF THE INVENTION

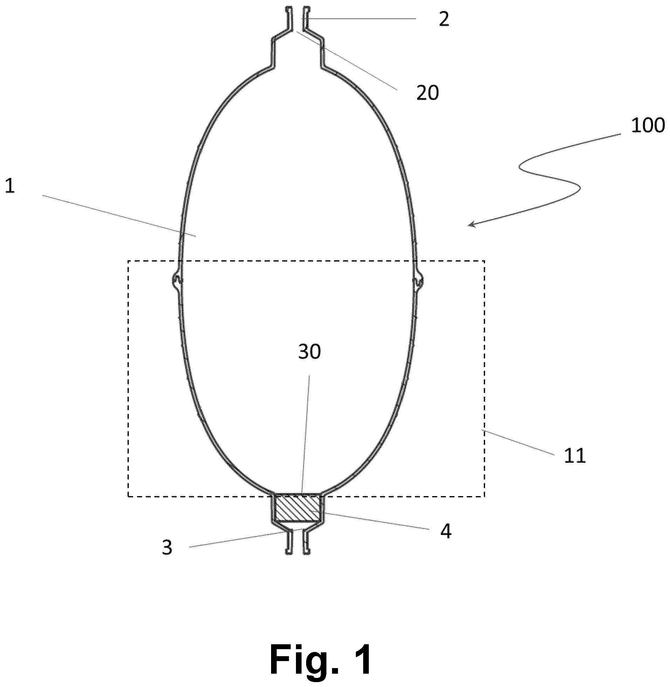

As shown in Figures 1 to 4, the device 100 of the invention, configured for separating by decantation a first waste phase and a second processed fatty tissue phase after mixing the fatty tissue with a washing solution, comprises a decantation chamber 1 comprising a decantation surface 11 where the fatty tissue and washing solution mixture is arranged and where separation by decantation takes place, and also comprises a waste extraction conduit 3 for extracting the waste phase, and a filter 4 for preventing the passage of the processed fatty tissue phase through the waste extraction conduit 3. The waste extraction conduit 3 is communicated with the decantation chamber 1 through an opening 30 of the decantation chamber 1, the filter 4 is housed in the waste extraction conduit 3, and when the device is in a waste extraction position or in a decantation and extraction position where both the decantation position and the extraction position coincide (like in the embodiments shown in the figures), the waste extraction conduit 3 is arranged below the decantation chamber 1.

In a preferred embodiment, the device further comprises a fatty tissue extraction conduit 2 communicated with the decantation chamber 1 through an opening 20 of the decantation chamber 1, the device being configured so that, in the waste decantation and extraction position, the fatty tissue and washing solution mixture cannot go through said fatty tissue extraction conduit 2. In one embodiment, this fatty tissue extraction opening and conduit 2 can also be used for introducing the fatty tissue to be processed and washing solution mixture together or separately into the decantation chamber 1.

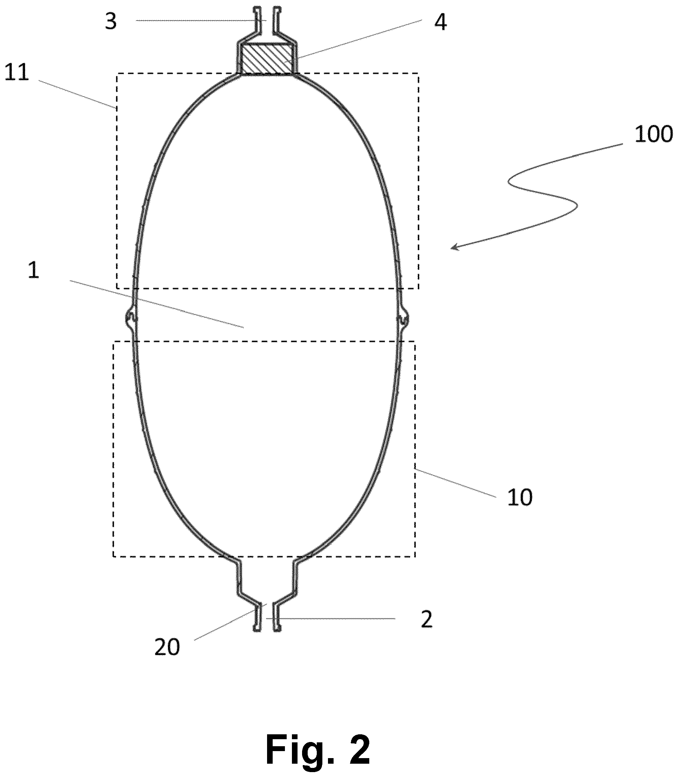

In a preferred embodiment, when the device is in the waste extraction position, the opening 20 of the decantation chamber 1 through which the fatty tissue extraction conduit 2 is communicated is arranged above the decantation surface 11, the device being configured for being reoriented once the waste phase is extracted, for example by means of a rotation, and arranged in a fatty tissue extraction position to allow extracting the fatty tissue phase through the fatty tissue extraction conduit 2, as shown in Figure 2. Preferably, the openings 20, 30 are arranged in distal positions inside the decantation chamber 1.

In one embodiment, the decantation surface 11 is concave or frustoconical and converges towards the opening 30 communicated with the waste extraction conduit 3. During separation by decantation, this concave or frustoconical surface 11 acts as a support surface for the fatty tissue and washing solution mixture, and once decantation has taken place, during the extraction of the waste phase, the actual curvature of the surface facilitates the extraction of the waste through the opening 30 communicated with the waste extraction conduit 3.

In a preferred embodiment, the decantation chamber 1 comprises an additional concave or frustoconical surface 10 converging towards the opening 20 communicated with the fatty tissue extraction conduit 2 and acting as a support surface for the fatty tissue in the fatty tissue extraction position.

Both the decantation surface 11 and the additional surface 10 are formed by inner walls of the decantation chamber, so depending on whether the device is in the waste phase decantation and extraction position or in the processed fatty tissue phase extraction position, part of the decantation surface 11 and the additional surface 10 may coincide.

These concave or frustoconical shapes converging towards the openings 20, 30 facilitate the collection of the waste phase and/or processed fatty tissue phase, and therefore improve the extraction efficiency, both from the viewpoint of the method and the amount of extracted material, and therefore the exploitation of the processed fatty tissue.

In a preferred embodiment, the additional surface 10 is in a distal position with respect to the decantation surface 11, such that for the device to transition from the waste extraction position to the fatty tissue extraction position, said device must be rotated about 180°. In a preferred embodiment, the openings 20, 30 are also arranged in distal positions. These arrangements and rotation of the device, in addition to consisting of a simple movement for the user, improve waste and processed fatty tissue extraction efficiency aided by the force of gravity.

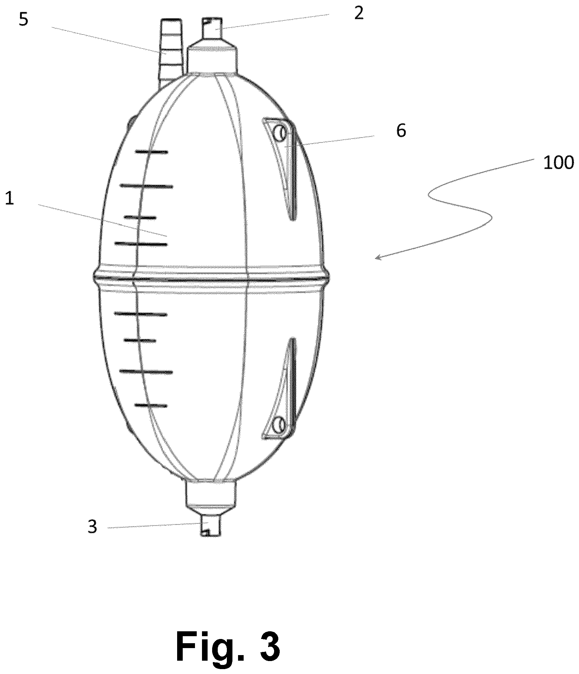

In a preferred embodiment, the decantation surface 11 and the additional surface 10 are substantially symmetrical to one another, preferably the decantation chamber 1 being in the shape of a prolate spheroid or similar to a rugby ball, as shown in Figure 3.

In terms of the capacity of the decantation chamber 1, in a preferred embodiment, the decantation chamber 1 of the device has a maximum capacity of 2000 ml, preferably between 100 ml and 1000 ml.

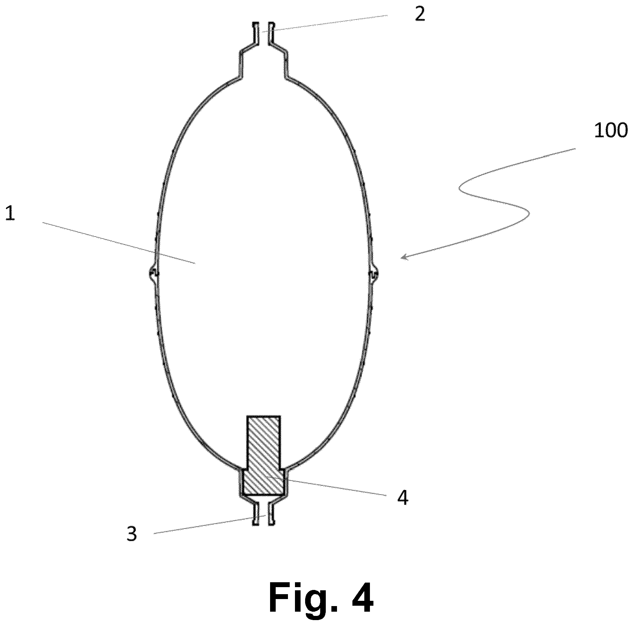

With respect to the filter 4 housed in the waste extraction conduit 3, it can have a conical, tubular, disk, or square shape. Depending on the shape, in some embodiments, part of the filter 4 may protrude into the decantation chamber 1, as shown in Figure 4.

In one embodiment, the device further comprises an opening 5 which is communicated with the decantation chamber 1 and allows the passage of air or gases from the decantation area, for example, from the air exiting during the introduction of unprocessed fatty tissue into the decantation area 1. In a preferred embodiment, the opening 5 is configured for coupling vacuum or suction-generating means during the introduction of unprocessed fatty tissue into the decantation chamber 1. In terms of where this opening 5 is located in the device, it is preferably located on the same side as or adjacent to opening 20. The fatty tissue to be processed is thereby prevented from seeping out through the opening 5 during the introduction of the tissue into the decantation area, simplifying the structure of the device.

In one embodiment, the fatty tissue conduit 2, and/or the waste extraction conduit 3, and/or the opening 5 are configured for being coupled to a valve, cannula, stopper, and/or syringe. This allows greater handling capacity for the user as they will be allowed to regulate and/or control the flow of the fluids to be introduced into and extracted from the decantation chamber 1, and to connect the device to other devices or elements necessary for carrying out liposuction, introducing washing solutions or tissue graft, and/or extracting the processed fatty tissue, allowing all this to be performed in a closed circuit. Some examples of valves which can be used in the invention include: Luer activated valves, drainage valves, fluid-level indicators, hemostasis valves, transfer valves, and pressure valves. Preferably, the valve is of the Luer activated or transfer type.

In a preferred embodiment, the device comprises a material which allows viewing the decantation process in the decantation chamber 1 and/or the waste and/or tissue extraction processes through the corresponding conduits 2, 3. The user is therefore able to view and differentiate processed adipose tissue from waste while each of them is being extracted.

In a preferred embodiment, the device comprises a material with a modulus of elasticity between 1600 MPa and 3000 MPa. This degree of elasticity prevents the device from collapsing if it is subjected to a vacuum, for example, to the vacuum used for introducing the fatty tissue 2 to be processed into the decantation chamber 1.

In a preferred embodiment, the polymer material is selected from polycarbonate, ASA, ABS, PS, SAN, COP, COC, or a mixture thereof. Preferably, this polymer material has a haze value of less than or equal to 1%.

In a preferred embodiment, the device further comprises at least one holding element 6, as illustrated in Figure 3. This element 6 allows greater handling capacity for the user while using the device in the different decantation or extraction positions. This holding element 6 can be a flange comprising a hole for housing a hook of an IV support or infusion supports, for example.

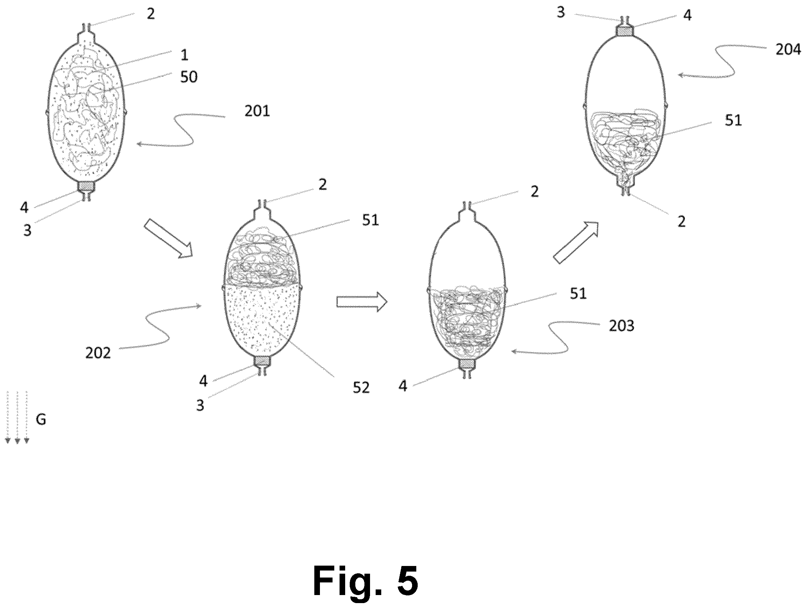

Another aspect of the invention relates to the method for processing fatty tissue of animal origin as schematically shown in Figure 5. The method comprises the following steps:
- a step 201 of mixing 50 an amount of fatty tissue of animal origin and a washing solution in a decantation chamber 1;
- a step 202 of separating by decantation a first waste phase 52 and a second processed fatty tissue phase 51 in the decantation chamber 1; as can be seen in Figure 5, the waste phase 52 is arranged below the fatty tissue phase 51 as it is denser than the fatty tissue phase 51;
- a step 203 of extracting the waste phase 52 from the decantation chamber 1 through a waste extraction conduit 3; and
- a step 204 of extracting the second processed fatty tissue phase 51 from the decantation chamber 1.

In the method of the invention, the waste phase 52 is filtered during the step of extracting the waste phase through the waste extraction conduit 3.

In Figure 5, step 203 illustrates the result following the extraction of the first waste phase 51. The arrows next to the letter G correspond to the direction of the force of gravity in the illustrated example.

In a preferred embodiment, the steps of the method are performed by means of a device such as the one described above, as illustrated in Figure 5, the device being in a decantation position in step 202, in a waste phase extraction position in step 203, and in an extraction position for extracting the second processed fatty tissue phase 51 in step 204. The method can be implemented in any of the embodiments or configurations of the device of the invention, the method thereby comprising the corresponding embodiment or configuration. All the features described for the device which are useful for the method are also valid for the method.

In a preferred embodiment, prior to step 201 of mixing 50, there is a step of introducing unprocessed fatty tissue of animal origin and a washing solution into the decantation chamber 1 through the fatty tissue conduit 20 and opening 2. After step 203 of extracting the first waste phase 52, the user will usually introduce more washing solution into the decantation chamber 1, based on the required washing needs of the tissue to be processed, step 202 of separating and step 203 of extracting the waste phase 52 being repeated as many times as necessary or required.

Another aspect of the invention relates to the use of the device of the invention for the processing of fatty tissue, preferably, for the processing of fatty tissue according to the method for processing of the invention.

## Claims

1. Device for the processing of fatty tissue of animal origin, the device being configured for separating by decantation a first waste phase and a second processed fatty tissue phase after mixing the fatty tissue with a washing solution, the device comprising a decantation chamber (1) comprising a decantation surface (11) where the fatty tissue and washing solution mixture is arranged and where separation by decantation takes place, and the device also comprising a waste extraction conduit (3) for extracting the waste phase and a filter (4) for preventing the passage of the processed fatty tissue phase through the waste extraction conduit (3), **characterized in that** the waste extraction conduit (3) is communicated with the decantation chamber (1) through an opening (30) of the decantation chamber (1), the filter (4) is housed in the waste extraction conduit (3), and when the device is in a waste extraction position, the waste extraction conduit (3) is arranged below the decantation chamber (1).

2. Device according to claim 1, comprising a decantation position coinciding with the waste extraction position.

3. Device according to claim 1 or 2, wherein the device comprises a fatty tissue extraction conduit (2) communicated with the decantation chamber (1) through an opening (20) of the decantation chamber (1), the device being configured so that, in the waste extraction position, the fatty tissue and washing solution mixture cannot go through said fatty tissue extraction conduit (2).

4. Device according to claim 3, wherein when the device is in the waste extraction position, the opening (20) of the decantation chamber (1) through which the fatty tissue extraction conduit (2) is communicated is arranged above the decantation surface (11), the device being configured for being reoriented and arranged in a fatty tissue extraction position to allow extracting the fatty tissue phase once the waste phase is extracted.

5. Device according to claim 3 or 4, wherein the decantation surface (11) is concave or frustoconical and converges towards the opening (30) communicated with the waste extraction conduit (3) and acts as a support surface for the fatty tissue and washing solution mixture during separation by decantation.

6. Device according to any of claims 3 to 5, wherein the decantation chamber (1) comprises an additional concave or frustoconical surface (10) that converges towards the opening (20) communicated with the fatty tissue extraction conduit (2) and acts as a support surface for the fatty tissue in the fatty tissue extraction position.

7. Device according to claim 6, wherein the additional surface (10) is in a distal position with respect to the decantation surface (11) such that for the device to transition from the waste extraction position to the fatty tissue extraction position, said device must be rotated 180°.

8. Device according to claim 7, wherein the decantation surface (11) and the additional surface (10) are substantially symmetrical to one another.

9. Device according to claim 8, wherein the decantation chamber (1) is in the shape of a prolate spheroid.

10. Device according to any of the preceding claims, wherein the filter (4) has a conical, tubular, disk, or square shape.

11. Device according to any of the preceding claims, comprising an opening (5) configured for coupling vacuum or suction-generating means.

12. Device according to any of the preceding claims, wherein the fatty tissue conduit (2) and/or waste extraction conduit (3) are configured for being coupled to a valve, cannula, stopper, and/or syringe.

13. Device according to any of the preceding claims, comprising a material which allows viewing the decantation process in the decantation chamber (1).

14. Device according to any of the preceding claims, comprising a material with a modulus of elasticity between 1600 MPa and 3000 MPa.

15. Device according to claim 13 or 14, comprising a polymer material selected from polycarbonate, ASA, ABS, PS, SAN, COP, COC, or a mixture thereof.

16. Device according to any of claims 13 to 15, wherein the polymer material has a Haze value of less than or equal to 1%.

17. Device according to any of the preceding claims, comprising at least one holding element (6).

18. Method for processing fatty tissue of animal origin, comprising the following steps:
- a step of mixing an amount of fatty tissue of animal origin and a washing solution in a decantation chamber (1);
- a step of separating by decantation a first waste phase and a second processed fatty tissue phase in the decantation chamber (1);
- a step of extracting the waste phase from the decantation chamber (1) through a waste extraction conduit (3); and
- a step of extracting the second processed fatty tissue phase from the decantation chamber (1),
**characterized in that** the waste phase is filtered during the step of extracting the waste phase through the waste extraction conduit (3).

19. Method for processing fatty tissue of animal origin according to claim 18, wherein the steps of the method are performed by means of a device according to any of claims 1 to 17.

20. Use of the device according to any of claims 1 to 17 for the processing of fatty tissue.
